(19) Europäisches Patentamt
European Patent Office
Office européen des brevets

(11) **EP 4 120 911 B1**

(12) **FASCICULE DE BREVET EUROPEEN**

(45) Date de publication et mention de la délivrance du brevet:
**01.05.2024 Bulletin 2024/18**

(21) Numéro de dépôt: **21717761.7**

(22) Date de dépôt: **16.03.2021**

(51) Classification Internationale des Brevets (IPC):
***A61B 5/11*** *(2006.01)* ***A61B 5/00*** *(2006.01)*

(52) Classification Coopérative des Brevets (CPC):
**A61B 5/1102; A61B 5/725;** A61B 5/6892;
A61B 5/7203; A61B 5/7225; A61B 2562/0219;
A61B 2562/0247; A61B 2562/046

(86) Numéro de dépôt international:
**PCT/EP2021/056667**

(87) Numéro de publication internationale:
**WO 2021/185825 (23.09.2021 Gazette 2021/38)**

(54) **DISPOSITIF ET PROCÉDÉ DE BALLISTOCARDIOGRAPHIE**

BALLISTOKARDIOGRAPHIEVORRICHTUNG UND VERFAHREN

BALLISTOCARDIOGRAPHY DEVICE AND METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.03.2020 FR 2002547**

(43) Date de publication de la demande:
**25.01.2023 Bulletin 2023/04**

(73) Titulaires:
• **Paris Sciences et Lettres**
**75006 Paris (FR)**
• **Ecole Pratique des Hautes Etudes**
**75014 Paris (FR)**

(72) Inventeur: **CATHELAIN, Guillaume**
**92130 Issy les Moulineaux (FR)**

(74) Mandataire: **Cornuejols, Marine Sophie**
**Cassiopi**
**230, avenue de l'Aube Rouge**
**34170 Castelnau-le-Lez (FR)**

(56) Documents cités:
**US-A1- 2009 054 742**

• **POSTOLACHE O ET AL: "Vital Signs Monitoring System Based on EMFi Sensors and Wavelet Analysis", INSTRUMENTATION AND MEASUREMENT TECHNOLOGY CONFERENCE PROCEEDINGS, 2007 IEEE, IEEE, PI, 1 mai 2007 (2007-05-01), pages 1-4, XP031182209, ISBN: 978-1-4244-0588-6**
• **RIKKY MULLER ET AL: "A 0.013 mm2, 5 $\mu$W , DC-Coupled Neural Signal Acquisition IC With 0.5 V Supply", IEEE JOURNAL OF SOLID-STATE CIRCUITS, IEEE, USA, vol. 47, no. 1, 1 janvier 2012 (2012-01-01), pages 232-243, XP011391118, ISSN: 0018-9200, DOI: 10.1109/JSSC.2011.2163552**
• **Mohammad Mojarradi ET AL: "Low-Cutoff, High-Pass Digital Filtering of Neural Signals", NASA Tech Briefs, 1 septembre 2004 (2004-09-01), XP055765371, Extrait de l'Internet: URL:https://ntrs.nasa.gov/api/citations/20 110020527/downloads/20110020527.pdf [extrait le 2021-01-15]**

**Description**

DOMAINE TECHNIQUE DE L'INVENTION

La présente invention vise un dispositif et un procédé de ballistocardiographie. Elle s'applique, notamment, à la ballistocardiographie, c'est-à-dire à la mesure non intrusive de l'activité cardiaque mécanique.

ÉTAT DE LA TECHNIQUE

La ballistocardiographie (BCG), une méthode de surveillance du rythme cardiaque sans contact, permet de mesurer les signes vitaux et de réduire la douleur et l'inconfort du patient en matière de surveillance. Elle utilise un accéléromètre placé sur le côté du matelas. Comme l'amplitude du signal est très faible, le circuit électronique frontal de conditionnement du signal a besoin d'un gain d'amplification élevé et est donc très sensible à la dérive de la composante continue et au bruit de fond.

Cependant, les filtres analogiques connus sont lents à se stabiliser, et le patient plus le lit constituent un système mécanique masse-ressort qui génère du bruit à sa fréquence de résonance. Par conséquent, le signal du ballistocardiogramme est susceptible de saturer pendant plusieurs secondes chaque fois que le patient se déplace.

Dans l'art antérieur, une amplification analogique est nécessaire pour obtenir de bonnes performances de détection. Elle est donc très sensible à la dérive de la composante continue qui se produit lors d'un changement de position du patient, par exemple de la position couchée sur le ventre à la position couchée sur le dos, et doit être filtrée. Cependant, pour mesurer l'activité respiratoire dont les composantes de basse fréquence sont d'environ 0,1 Hz, les filtres passe-haut habituels sont lents à se stabiliser et le signal du ballistocardiogramme peut saturer pendant plusieurs dizaines de secondes. L'ajout de composantes non linéaires, par exemple des diodes de commutation dans la boucle de rétroaction de l'amplificateur, peut aider à réduire la durée de stabilisation de l'amplificateur, c'est-à-dire la durée avant la fin de la saturation, pour quelques secondes seulement. De plus, le patient et son lit peuvent être considérés comme un système masse-ressort qui génère du bruit à sa fréquence de résonance. Des bruits environnants, par exemple la marche des infirmières, les appareils de ventilation ou pendant le transport dans l'unité de soins intensifs pédiatriques, peuvent également se produire. Pour les mêmes raisons de saturation, ces bruits répétitifs doivent être filtrés, sinon la durée de surveillance risque d'être réduite.

La publication scientifique de Postolache O. et al. Intitulée « Vital Signs Monitoring System Based on EMFi Sensors and Wavelet Analysis » décrit un système de surveillance des signes vitaux basé sur des capteurs EMFi (capteurs électromécaniques à film) et une analyse en ondelettes. La publication scientifique de Rikky Muller et al. Intitulée « A 0.013mm2, 5μW, DC-Coupled Neural Signal Acquisition IC with 0.5 V Supply » présente un système d'acquisition de signaux neuronaux couplé au courant continu qui utilise une architecture à forte intensité numérique pour réduire la surface du système et permettre son fonctionnement à partir d'une alimentation de 0,5 V. La publication scientifique de Mohammad Moiarradi et al. Intitulée « Low-Cutoff, High-Pass Digital Filtering of Neural Signals » décrit un filtrage numérique passe-haut à faible coupure des signaux neuronaux. Selon ce document, ce filtrage numérique permet de surmonter les inconvénients du filtrage par résistance et condensateur. US 2009/0054742 décrit un appareil pour la détection, le traitement et la communication de signal. Cet appareil pour surveiller un ou plusieurs paramètres biologiques comprend au moins deux couches, chaque couche comprenant un substrat flexible. Les couches comprennent au moins deux capteurs pour détecter chaque paramètre biologique.

PRÉSENTATION DE L'INVENTION

La présente invention vise à remédier à tout ou partie de ces inconvénients.

À cet effet, selon un premier aspect, la présente invention vise un dispositif de ballistocardiographie qui comporte :

- un support pour une personne,
- une pluralité de capteurs de pression ou d'accélération fournissant, chacun, un signal analogique représentatif d'une pression ou d'une accélération mesurée en un point du support ou sur le corps de la personne,
- un multiplexeur configuré pour recevoir une pluralité de signaux analogiques en provenance de capteurs et pour fournir, en sortie, un signal successivement représentatif des signaux en entrée et
- un amplificateur dont une entrée est reliée à la sortie du multiplexeur et muni, sur son autre entrée, d'un filtre mixte comportant un convertisseur analogique numérique convertissant le signal sortant du multiplexeur en données numériques, un filtre numérique agissant sur les données numériques et un convertisseur numérique analogique convertissant les données numériques filtrées en un signal analogique injecté sur cette autre entrée de l'amplificateur.

Grâce à ces dispositions, l'état et les coefficients du filtre numérique peuvent être modifiés suffisamment rapidement pour que chaque capteur soit associé, successivement, à un filtrage numérique spécifique. Le dispositif peut ainsi mettre en oeuvre des centaines, voire des milliers de capteurs et réaliser une analyse plus fine et à moindre coût des données recueillies. De plus, le taux d'amplification peut être plus élevé, car la composante continue peut-être mieux filtrée. Chacun des signaux à traiter peut donc être de meilleure qualité et améliorer encore l'analyse réalisée. Enfin, chaque filtre numérique utilisé peut avoir un ordre plus élevé que des filtres analogiques. L'invention permet ainsi de réaliser une cartographie cardiaque plus précise que ce que permettaient les dispositifs de ballistocardiographie connus.

Dans des modes de réalisation, le dispositif comporte, pour chaque capteur, une mémoire des données numériques sortant successivement du convertisseur analogique numérique lorsque le signal sortant de ce capteur est fourni en sortie du multiplexeur, et le filtre comporte un processeur qui traite les données successivement mémorisées pour appliquer un filtrage de ces données et fournir une donnée numérique qui est convertie par le convertisseur numérique analogique.

Grâce à ces dispositions, les signaux issus des différents capteurs sont traités indépendamment, mais en tenant compte de leur évolution pour éliminer la dérive de la composante continue et stabiliser le filtrage appliqué.

Dans des modes de réalisation, le filtre numérique est une cascade de filtres biquadratiques, autrement appelée filtres biquadratiques en cascade.

C'est un filtre efficace, très facilement implémentable dans des systèmes embarqués tels que les microcontrôleurs.

Dans des modes de réalisation, la fréquence d'échantillonnage du convertisseur analogique numérique est égale au produit du nombre de capteurs par un nombre compris entre 128 et 512.

Grâce à ces dispositions, les fréquences cardiaques et de respiration sont finement détectées.

Dans des modes de réalisation, la pluralité de capteurs constitue au moins une matrice de capteurs de pression.

Grâce à ces dispositions, des centaines, voire des milliers, de capteurs sont mis en oeuvre et la cartographie ballistocardiographique ou cardio-respiratoire possède une résolution élevée.

Dans des modes de réalisation, le gain de l'amplificateur est supérieur à 10.

Les vibrations détectées sont ainsi de très faible amplitude, ce qui augmente la précision du dispositif.

Selon un deuxième aspect, la présente invention vise un dispositif de surveillance d'un patient pendant une opération chirurgicale.

Selon un troisième aspect, la présente invention vise une application d'un dispositif objet de l'invention à la visualisation de l'onde de pouls et de la contraction des ventricules.

Cette application permet de réaliser un scanner simplifié de fonctions vitales, par exemple visualisation de l'onde de pouls et de la contraction des ventricules, à moindre coût et en temps réel au cours d'une opération ou pendant la surveillance d'un patient.

Selon un quatrième aspect, la présente invention vise un procédé de ballistocardiographie mettant en oeuvre un dispositif objet de la présente invention, qui comporte les étapes suivantes :

- une pluralité de captures de pression ou d'accélération en différents points d'un support d'une personne ou sur le corps de la personne, fournissant, pour chaque point de capture, un signal analogique représentatif d'une pression ou d'une accélération mesurée en un point du support ou sur le corps de la personne,
- un multiplexage de la pluralité de signaux analogiques et
- une amplification du signal multiplexé auquel est soustrait un signal filtré par un filtre mixte comportant un convertisseur analogique numérique convertissant le signal multiplexé en données numériques, un filtre numérique agissant sur les données numériques et un convertisseur numérique analogique convertissant les données numériques filtrées en un signal analogique.

Dans des modes de réalisation, le procédé objet de l'invention comporte, de plus, une étape d'estimation de la composante continue et une étape d'initialisation du filtre avec la composante continue estimée.

Grâce à ces dispositions, la durée de stabilisation du signal filtrée est très réduite.

Dans des modes de réalisation, le procédé objet de l'invention comporte, de plus :

- une étape de détection d'une vibration dans le signal amplifié,
- une étape de segmentation du signal de vibration,
- une étape de transformation, dans le domaine fréquentiel, de segments du signal,
- une étape d'obtention d'un maximum du signal, dans le domaine fréquentiel,
- une étape de configuration d'un filtre coupe-bande comportant le maximum obtenu et
- une étape de filtrage du signal amplifié avec le filtre coupe-bande.

Grâce à ces dispositions, le bruit, par exemple engendré par les mouvements du patient, est extrait du signal traité et visualisé.

Les buts, avantages et caractéristiques particulières du procédé et des applications objets de la présente invention étant similaires à ceux du dispositif objet de la présente invention, ils ne sont pas rappelés ici.

BRÈVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :

- La figure 1 représente, schématiquement, un mode de réalisation particulier du dispositif objet de l'invention,
- La figure 2 représente, schématiquement, un circuit électronique frontal de conditionnement du signal issu de capteurs,
- La figure 3 représente, schématiquement, un exemple d'un filtre IIR de quatrième ordre,
- La figure 4 représente une distribution des pôles d'un filtre IIR quantifiés sur six bits,
- La figure 5 représente une quantification d'un pôle d'un filtre IIR quantifié sur 64 bits à 32 bits,
- La figure 6 représente, sous forme d'une courbe fonction de la fréquence, un effet de la quantification et de la rectification de la bande passante d'un filtre IIR,
- La figure 7 représente des réponses en fréquence de l'équivalent passe-bas des filtres conçus et du filtre RC analogique typique,
- La figure 8 représente des étapes d'un pseudoalgorithme d'identification et de filtrage de la fréquence du bruit,
- La figure 9 est une illustration de la suppression de la dérive de la composante continue avec une amplification de 21 dB,
- La figure 10 représente une comparaison des réponses en fréquence pour un gain de 21 dB, IIR quantifié par rapport à des points expérimentaux,
- La figure 11 représente un effet de l'initialisation de l'état du filtre sur la durée de stabilisation,
- La figure 12 représente une durée de stabilisation pour différentes initialisations de l'état du filtre avec une amplification de 21 dB,
- La figure 13 représente un exemple d'une impulsion de BCG et de son spectre de fréquence unilatéral calculé par un microcontrôleur,
- La figure 14 représente, en amplitudes, la réponse fréquentielle de différents filtres,
- La figure 15 représente, en phases, la réponse fréquentielle de différents filtres,
- La figure 16 représente la réponse temporelle du filtre et de l'amplificateur dans la configuration FIR,
- La figure 17 représente la réponse temporelle du filtre et de l'amplificateur dans la configuration IIR une section,
- La figure 18 représente la réponse temporelle du filtre et de l'amplificateur dans la configuration IIR cinq sections et
- La figure 19 représente la réponse temporelle du filtre et de l'amplificateur dans la configuration FIR avec initialisation de l'état du filtre.

DESCRIPTION DES MODES DE RÉALISATION

La présente description est donnée à titre non limitatif, chaque caractéristique d'un mode de réalisation pouvant être combinée à toute autre caractéristique de tout autre mode de réalisation de manière avantageuse.

On note dès à présent que les figures ne sont pas à l'échelle.

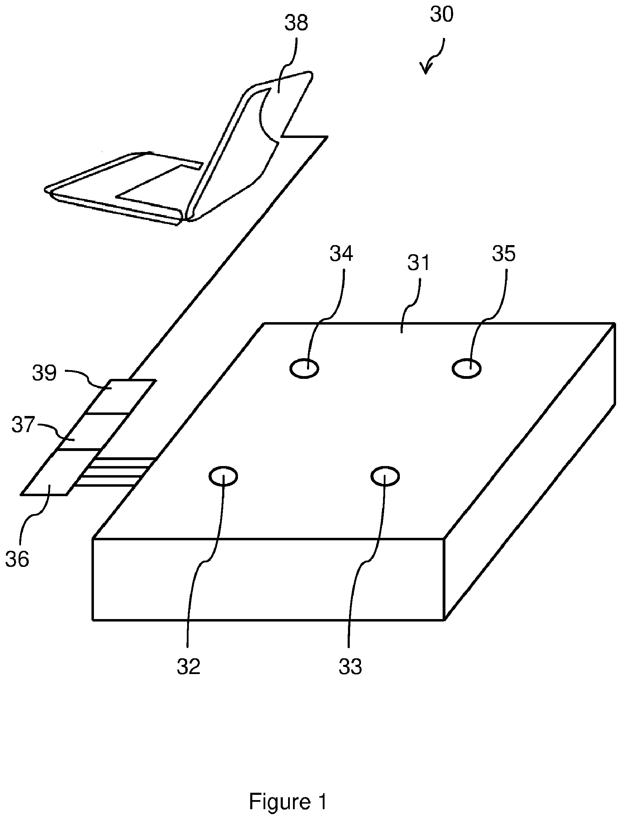

On utilise, dans la description, indifféremment les termes de processeur et de microcontrôleur. On observe, en figure 1, une vue schématique d'un premier mode de réalisation 30 d'un dispositif objet de la présente invention.

On observe, en figure 1, un support 31 pour une personne (non représentée), des capteurs 32 à 35 disséminés dans ou sur le support 31, un circuit électronique frontal 36 de conditionnement et d'amplification des signaux issus des capteurs 32 à 35, un convertisseur analogique numérique 37 des données sortant du circuit 36, un microcontrôleur 39 et un ordinateur 38 de traitement des données.

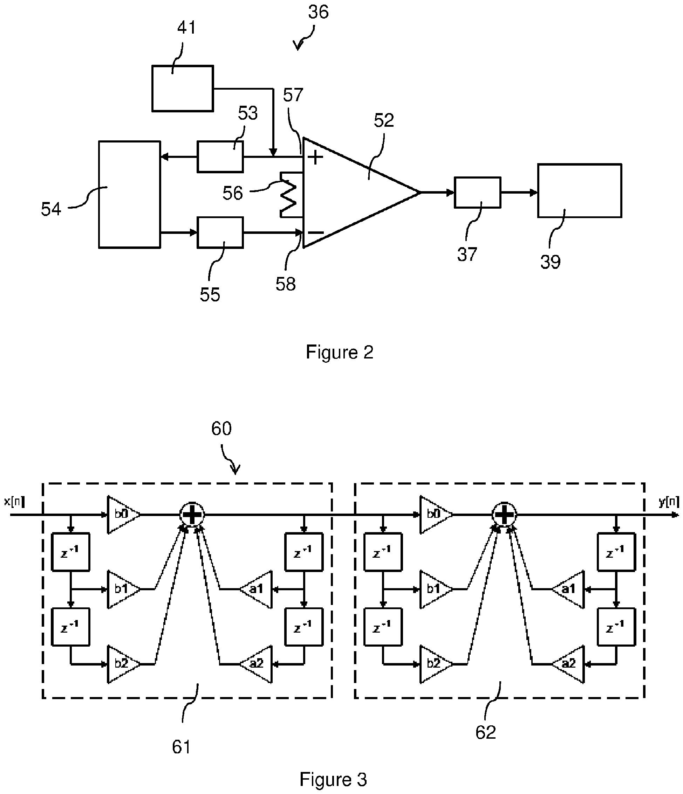

Le support 31 est, par exemple un lit, un matelas, un coussin ou un siège. Les capteurs 32 à 35 sont, par exemple, des capteurs de pression, éventuellement sous forme de matrices comme décrit plus bas, ou des accéléromètres. Un mode de réalisation particulier du circuit 36 est représenté en figure 2. L'ordinateur 38 de traitement des données réalise, par exemple, une cartographie cardiaque ou pulmonaire de la personne, en fonction des signaux numériques sortant du convertisseur analogique numérique 37, selon des algorithmes connus.

Le circuit frontal 36 identifie la fréquence de résonance du système, filtre la dérive de la composante continue et le bruit d'amortissement, et amplifie fortement le signal sans saturation, ou avec une durée de saturation réduite, après le mouvement du patient. Le circuit frontal 36 comporte, dans le mode de réalisation illustré en figure 2, un multiplexeur 41 des signaux analogiques provenant des capteurs 32 à 35, suivi d'un amplificateur 52 d'instrumentation dont la sortie est reliée au convertisseur analogique numérique 37. L'amplificateur 52 présente un gain très élevé, par exemple supérieur à 10, voire à 100. Le convertisseur analogique numérique 37 est précédé d'un filtre anti-repliement intégré dans le convertisseur analogique numérique 37.

Le signal multiplexé issu du multiplexeur 41 est fourni à une entrée (ici l'entrée positive 57) de l'amplificateur 52 et à un convertisseur analogique numérique 53. Un filtre numérique 54 filtre le signal numérisé et un convertisseur numérique analogique 55 convertit le signal filtré numériquement et le délivre à l'autre entrée (ici l'entrée négative 58) de l'amplificateur 52. Une résistance 56 commande le gain de l'amplificateur 52, entre les entrées de l'amplificateur opérationnel 52.

Le processeur de signal numérique (ou DSP pour « Digital Signal Processor ») , qui réalise le filtre numérique 54 est un STM32L476 (marque déposée) de 32 bits, monté sur une carte Nucleo-L476RG (marque déposée), dont les canaux des convertisseurs analogiques-numériques (ou ADC pour Analog to Digital Converter) et des convertisseurs numériques analogiques (ou DAC pour Digital to Analog Converter) ont une résolution de 12 bits et une fréquence d'échantillonnage fs comprise entre 128 et 512 Hz, par exemple de 256 Hz par capteur.

La fréquence du multiplexeur est donc N x fs, d'au moins 130 kHz pour atteindre la résolution spatio-temporelle minimale pour 1024 capteurs (fs = 128 Hz et N = 1024 capteurs) et jusqu'à 2 MHz pour la résolution spatio-temporelle maximale (fs = 512 Hz et N = 4096 capteurs). La résolution du quantificateur est de 8 à 16 bits, typiquement 12 bits.

Le microcontrôleur 39 démultiplexe les signaux filtrés en sortie du convertisseur analogique numérique 37 et conditionne ces signaux en matrice puis les envoie à l'ordinateur 38. L'ordinateur 38 affiche ensuite les matrices sous forme d'une vidéo puis compresse éventuellement ces matrices. Le système d'exploitation ARM MBED (marque déposée) a été utilisé avec la bibliothèque DSP de CMSIS (marque déposée). Un port série universel (ou USB pour Universal Serial Bus) est utilisé pour la communication série avec un ordinateur et l'alimentation du circuit.

Le capteur est un accéléromètre LIS344ALH (marque déposée) avec une faible densité de bruit de 50 $\mu$g/$\sqrt{\text{Hz}}$ et une haute sensibilité de 0,66 V/g. Il est monté sur une carte d'évaluation STEVAL-MKI015V1 (marque déposée), et les broches FS, PD et ST sont mises à la terre. Il est alimenté par une alimentation unique de 3,3 V et mesure des accélérations de +/- 2 g dans cette configuration. Il est orienté de telle sorte que l'axe Z soit dans la direction de la gravité.

Le circuit mixte frontal 36 de conditionnement de signaux possède une architecture de signal mixte, c'est-à-dire une topologie où un signal analogique est numérisé par un convertisseur analogique numérique ADC, filtré sur un processeur de traitement du signal DSP puis synthétisé par un convertisseur numérique analogique DAC. De tels circuits présentent les mêmes avantages que les filtres numériques, par exemple une bande de fréquence de transition nette ou une durée de stabilisation très réduite, et peuvent être associés à un amplificateur analogique.

Dans le cas du BCG, les composantes indésirables du signal d'accélération analogique doivent être filtrées avant l'amplification et la numérisation. L'architecture conçue, illustrée à la figure 2, élimine ces composantes indésirables et amplifie le signal résultant à l'aide d'un amplificateur d'instrumentation.

Le signal résultant est exprimé par l'équation 1, où G représente le gain de l'amplificateur et f représente le filtre numérique.

$$V_{out} = G . [Vin - f(Vin)] \qquad \text{(équation 1)}$$

Lors de la synthèse du signal de retour, une saturation est mise en oeuvre afin de respecter l'équation 2.

$$0 < G . [Vin - f(Vin)] + Vref < 3.3 \qquad \text{(équation 2)}$$

Conception et mise en oeuvre des filtres numériques : afin d'éliminer les composantes indésirables du signal, deux types de filtres linéaires numériques ont été mis en oeuvre dans des modes de réalisation du dispositif objet de l'invention. Il s'agit de filtres à réponse impulsionnelle finie (FIR) et les filtres à réponse impulsionnelle infinie (IIR) du deuxième ordre, représentés en figure 3 et communément appelés filtres biquadratiques en cascade (en anglais « cascade of biquad filters »). Les filtres FIR et filtres biquadratiques sont respectivement définis par l'équation 3 et l'équation 4, où n est l'indice d'échantillonnage, x et y sont les signaux d'entrée et de sortie, N est le nombre de coefficients, {a_n} et {b_n} sont les coefficients de rétroaction et de réaction.

$$y[n] = b_0 . x[n] + \cdots + b_{N-1} . x[n - N + 1] \qquad \text{(équation 3)}$$

$$y[n] = b_0 . x[n] + b_1 . x[n - 1] + b_2 . x[n - 2] + a_1 . y[n - 1] + a_2 . y[n - 2] \qquad \text{(équation 4)}$$

Par convention, $a_0$ est fixé à 1.

On observe, en figure 3, un exemple d'un filtre IIR de quatrième ordre 60, cascade de deux filtres biquadratiques 61 et 62, tel qu'implémenté par la bibliothèque DSP de CMSIS.

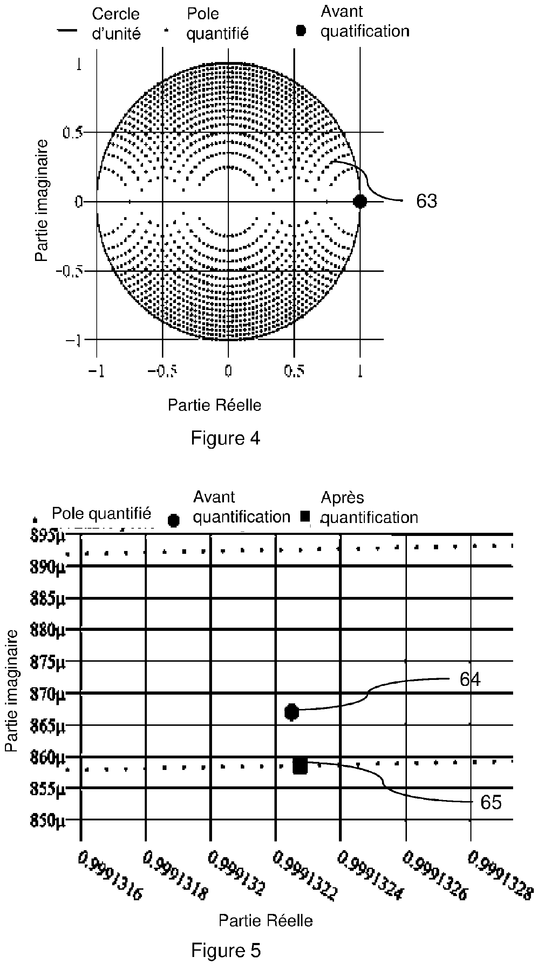

Les coefficients du filtre sont calculés à l'aide de la bibliothèque Python scipy.signal (marques déposées) sur un ordinateur 64 bits. Ils doivent être quantifiés à des coefficients de 32 bits avant d'être implémentés sur le processeur de traitement du signal DSP, ce qui peut générer des erreurs, en particulier pour les filtres IIR avec des filtres à fortes contraintes et une fréquence de coupure très basse. Par exemple, les figures 4 et 5 illustrent la quantification des pôles pour un filtre de Butterworth de deuxième ordre avec une fréquence de coupure de 0,05 Hz et un taux d'échantillonnage de 256 Hz.

En figure 4, la distribution des pôles 63 quantifiés sous six bits est représentée selon un axe horizontal représentant leur partie réelle et un axe vertical représentant leur partie imaginaire.

En figure 5, on observe la quantification d'un pôle 64 de 64 bits, avant quantification, en un pôle 65 de 32 bits, après quantification. Chacun de ces pôles est représenté selon un axe horizontal représentant sa partie réelle et un axe vertical représentant sa partie imaginaire.

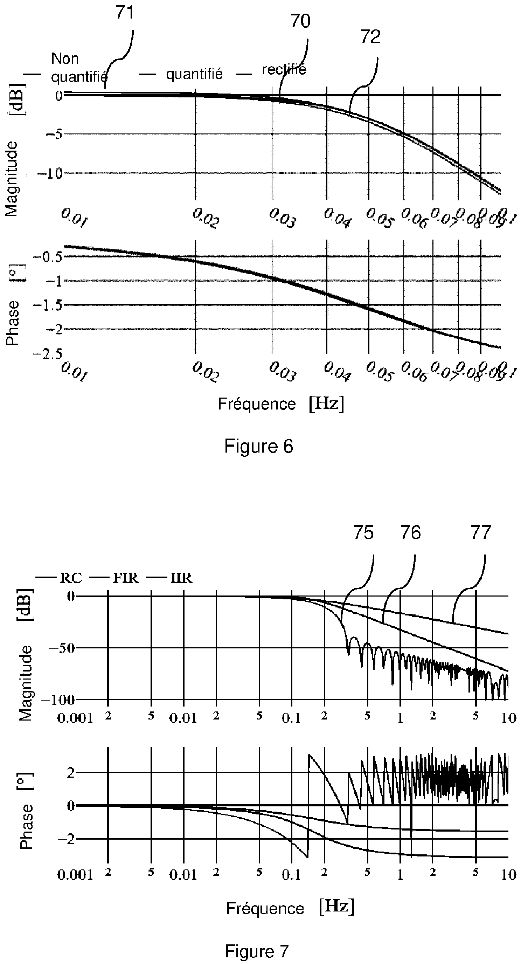

Cette quantification de coefficient est susceptible de modifier la réponse en fréquence du filtre, par exemple le gain de bande passante comme illustré à la figure 6 en utilisant le même filtre que celui des figures 4 et 5. Après quantification, le gain de la bande passante a été systématiquement corrigé à 0 dB à l'aide de l'équation 5.

$$b'_n = b_n \frac{\sum a_i}{\sum b_j} \qquad \text{(équation 5)}$$

On observe, en figure 6, avec un axe horizontal représentant les fréquences et deux axes verticaux représentant, en haut, les amplitudes et, en bas, les phases, un signal 70 non quantifié, un signal 71 quantifié et un signal 72 rectifié. La figure 6 représente ainsi l'effet de la quantification et de la rectification de la bande passante.

La durée de stabilisation des filtres dépend de l'initialisation. L'état du filtre est généralement initialisé à zéro : le signal est donc causal. Cependant, le capteur a une position de repos qui est connue ou peut être estimée. Dans le cas de l'accéléromètre décrit par ailleurs, on peut supposer qu'il est posé à plat sur le matelas qui est lui-même horizontal : l'axe z devrait voir une valeur de décalage d'environ +1 g, soit 2,31 V dans ce réglage. Le filtre est initialisé en considérant que le signal est de 2,31 V avant de commencer l'enregistrement.

Concernant la suppression de la dérive de la composante continue, celle-ci représente une composante souvent indésirable du signal physiologique. Il s'agit d'une composante à basse fréquence qui interdit une amplification à gain élevé. En ballistocardiographie, le déplacement de la composante continue est un décalage de tension dépendant de la position du corps et du poids dans le lit. Par exemple, lors d'un changement de position du patient, l'orientation de l'accéléromètre et la projection de la gravité sur les axes de l'accéléromètre changent. Les inventeurs ont observé que cette valeur de décalage se situe généralement entre 0,1 et 0,5 g, pour laquelle l'amplificateur saturerait avec G > 3 dB.

La respiration, dont la fréquence varie de 0,5 à 1,0 Hz en néonatologie et jusqu'à 0,1 Hz en gériatrie, peut être considérée soit comme une partie de la dérive de la composante continue, soit comme une composante utile du signal. Dans le mode de réalisation décrit ici, la respiration n'est pas filtrée, par conséquent la fréquence de coupure du filtre de base est fixée à 0,05 Hz et f est un filtre passe-bas (Équation 1).

Dans le cas du FIR, la méthode de la fenêtre est appliquée avec une fenêtre de Kaiser de 40 dB d'atténuation minimale dans la bande passante et une largeur de bande de transition de 0,1 Hz. Le filtre FIR résultant possède 5716 coefficients.

Dans le cas du FIR, un filtre de Butterworth de deuxième ordre, choisi en raison de sa réponse maximale plate dans la bande passante, est conçu et répété deux fois afin d'accentuer la transition. Dans le mode de réalisation décrit ici, le filtre choisi est un filtre IIR du quatrième ordre, car le filtre FIR est coûteux en calculs pour le microcontrôleur 54.

La figure 7 illustre les réponses des filtres FIR et IIR conçus en équivalent passe-bas. Elles sont comparées à un filtre typique à résistance-condensateur (RC) du premier ordre avec la même fréquence de coupure.

On observe, en figure 7, les réponses en fréquence de l'équivalent passe-bas des filtres FIR, réponse 75, IIR, réponse 76 et d'un filtre RC analogique typique, réponse 77. On note que les axes sont identiques à ceux de la figure 6, mais avec une étendue de la plage de fréquences plus importante.

Un gain de 21 dB est fixé dans ce mode de réalisation. Le filtre passe-haut équivalent a finalement le numérateur et les dénominateurs suivants dans le tableau 1. Il est défini par l'équation 6, l'équation 7 et l'équation 8.

$$V_{out} = g(V_{in}) \qquad \text{(équation 6)}$$

$$a'_n = a_n \qquad \text{(équation 7)}$$

$$b'_n = G.(a_n - b_n) \qquad \text{(équation 8)}$$

| a0 | 1 |
|----|---|
| a1 | -1.9982646 |
| a2 | 0.99826604 |
| b0 | 11.183295 |
| b1 | -22.3472 |
| b2 | 11.163904 |

Numérateurs a0, a1 et a2 et dénominateurs b0, b1, b2 du filtre passe-haut équivalent.

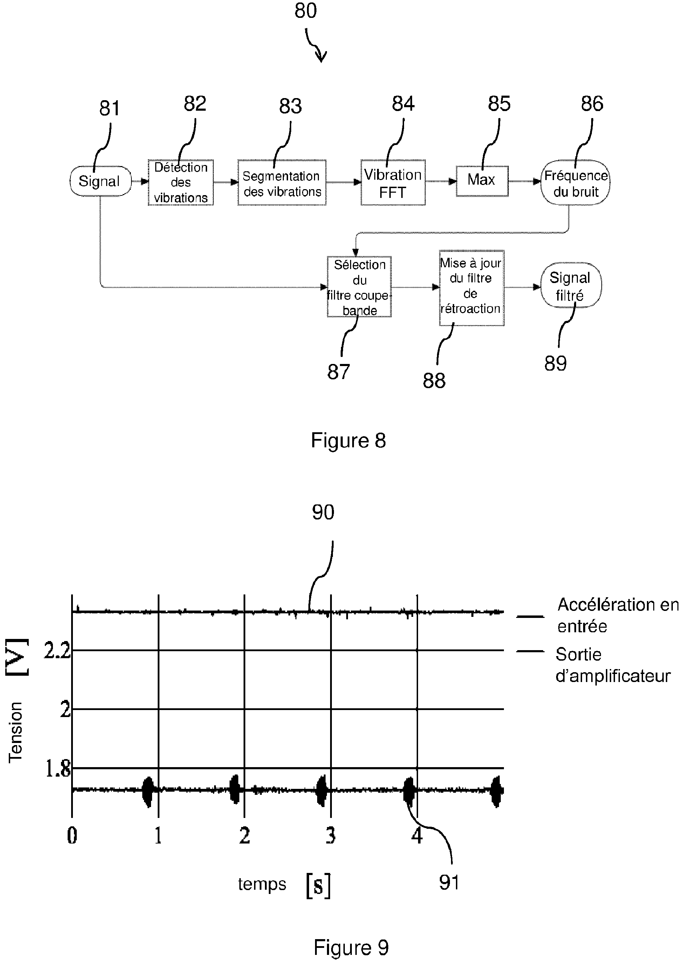

Concernant l'identification et le filtrage des fréquences de bruit, la fréquence du bruit est identifiée et filtrée à l'aide de l'algorithme 80 intégré illustré en figure 8.

On observe, en figure 8, une entrée 81 du signal amplifié, une étape de détection de vibration 82, une étape de segmentation de vibration 83, une étape de transformation de Fourier rapide (ou FFT pour « Fast Fourier Transform ») 84, une étape d'extraction de maximum 85 qui fournit la fréquence du bruit 86, une étape de sélection de filtre coupe-bande (« notch filter » en anglais) 87, une étape de mise à jour du filtre de réaction 88 et une étape de sortie du signal filtré 89.

L'étape de détection des vibrations 82 est paramétrée par une valeur limite de tension, ci-après dénommée « seuil », au-delà duquel la vibration est détectée. La vibration commence lors du dépassement, par le signal amplifié de cette valeur limite de tension, et possède une durée considérée comme fixe.

La densité spectrale de puissance de la vibration segmentée est calculée par le microcontrôleur. Son maximum est situé à la fréquence du bruit, de sorte qu'elle se situe en dehors de la gamme de fréquences du BCG, sinon les battements de coeur du signal BCG seraient déformés ou filtrés.

Plusieurs filtres coupe-bande, avec des facteurs de qualité Q=0,707, ont été préliminairement calculés à l'aide de la bibliothêque scipy.signal en dehors du microcontrôleur. Le filtre coupe-bande dont la fréquence est la plus proche de la fréquence du bruit est sélectionné, et ajouté au filtre de base de l'IIR.

Dans cette section, les résultats de l'expérience sont détaillés : le filtrage de la dérive de la composante continue, la comparaison de la durée de stabilisation et l'identification et le filtrage du bruit sont analysés. Les signaux bruts présentés ne sont pas post-traités.

Filtrage de la composante continue

Le filtre IIR réussit à filtrer la composante continue, et celle-ci est éliminée par l'amplificateur. À l'état d'équilibre, l'amplitude maximale crête à crête des impulsions est dix fois plus élevée à la sortie qu'à l'entrée. La figure 9 est une illustration du signal avant et après l'amplificateur avec une amplitude d'impulsion inférieure au niveau de bruit, avec une amplification de 21 dB.

On observe, en figure 9, une courbe 90 en sortie de capteur et une courbe 91 d'accélération amplifiée. L'axe horizontal représente le temps, en seconde et l'axe vertical représente le voltage. On observe qu'un pouls, de fréquence d'environ 60 hertz est extrait du signal bruité initial, malgré les défauts du signal initial.

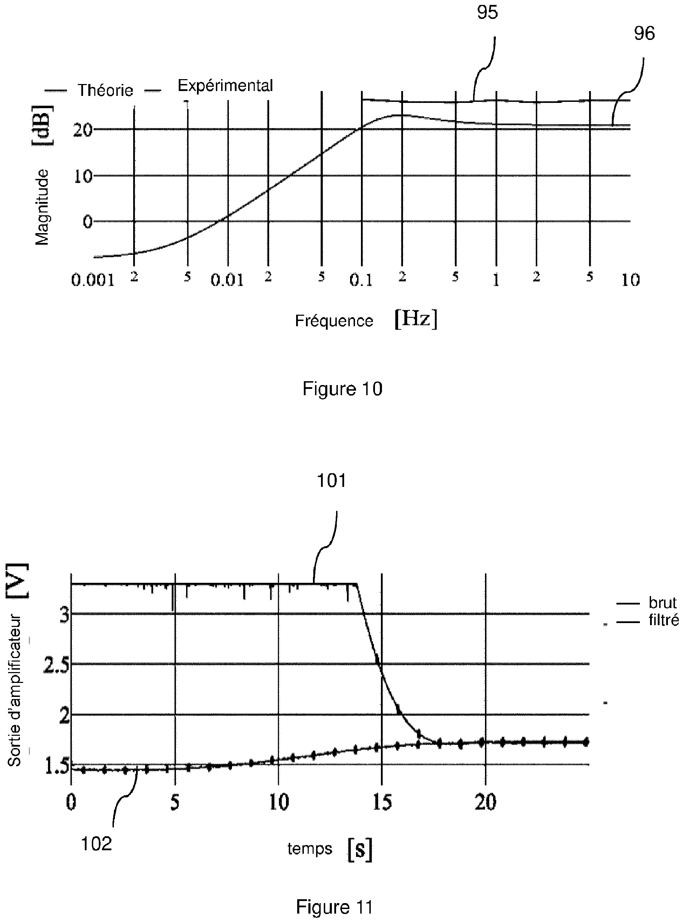

Ce filtre permet de générer des vibrations à différentes fréquences constantes et de comparer les accélérations mesurées avant et après le filtrage et l'amplification par leurs valeurs moyennes quadratiques. La réponse 95 en fréquence mesurée expérimentalement est comparée à la réponse théorique 96 de ce filtre, en figure 10, pour le filtre IIR quantifié, avec un gain de 21dB.

Comparaison des durées de stabilisation

Les signaux ont été enregistrés à différents niveaux d'initialisation. La figure 11 est une superposition de deux signaux à des moments différents, avec une initialisation décalée de 0 V, courbe 101, et 2,31 V, courbe 102. On observe que lorsque l'état du filtre est initialisé à 0 V, l'amplificateur peut saturer pendant des dizaines de secondes, ce qui n'est pas le cas si le décalage est bien estimé.

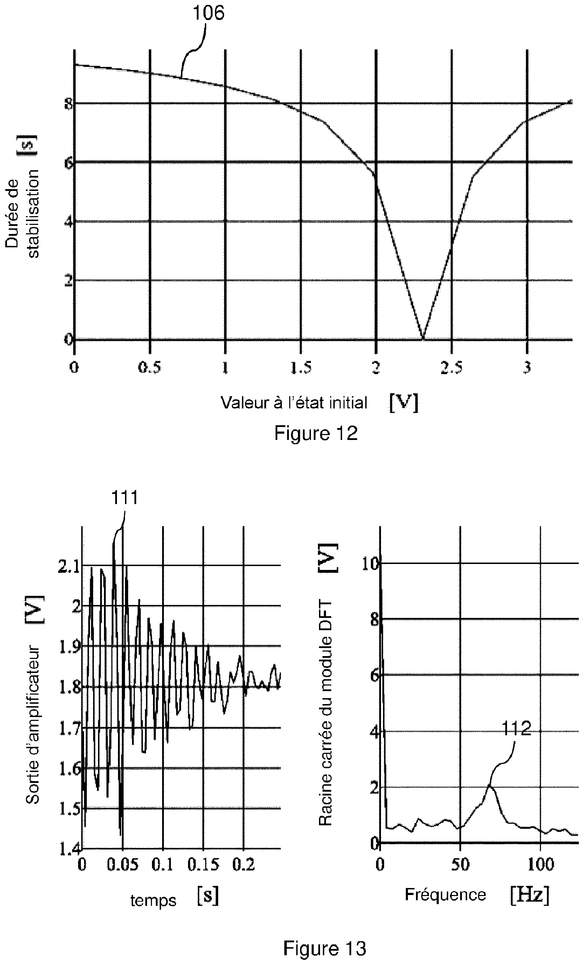

La durée de stabilisation, en ordonnées, dépend de l'initialisation du décalage, en abscisse, comme l'illustre la courbe 106 en figure 12, avec une amplification de 21 dB.

Identification des bruits

Le seuil et la durée ont été fixés respectivement à 1,5 % et 0,25 s de la dérive de la composante continue initiale. Le bruit était généré par des vibrations pulsées, c'est-à-dire le BCG simulé. La fréquence 112 du bruit 111 déterminée par le microcontrôleur est d'environ 68 Hz, comme on peut le voir sur la figure 13. Il s'agit de la fréquence de résonance du système d'amortissement.

Un filtre coupe-bande a ensuite été sélectionné par l'algorithme illustré par la figure 8 pour filtrer la fréquence de bruit identifiée.

En conclusion, la présente invention met en oeuvre un nouveau circuit frontal de conditionnement de signal qui identifie la fréquence de résonance du système, filtre la dérive de la composante continue et le bruit d'amortissement, et amplifie le signal BCG sans saturation après le mouvement du patient.

Dans la description ci-dessus, le gain d'amplification est de 21 dB pour illustrer le principe du circuit frontal BCG. Cependant, l'invention ne se limite pas à ce niveau de gain, notamment lorsqu'un circuit électronique spécifique avec un plan de masse adéquat est utilisé.

Par rapport aux instruments habituels du BCG, ce circuit frontal de conditionnement décrit ci-dessus à une durée de stabilisation plus faible et une transition de fréquence plus nette. Il ouvre des possibilités d'applications médicales, en temps réel et à faible coût.

On décrit, ci-dessous, l'intégration de ce circuit frontal dans un dispositif de BCG. On rappelle que les fréquences de 0,05 Hz à 25 Hz comportent les phénomènes cardiaques et respiratoires.

Le but de ce dispositif est d'établir une cartographie cardio-respiratoire de la surface corporelle par multiplexage temporel d'un filtre analogique à boucle de rétroaction numérique.

Un montage de filtre mixte 36 tel que décrit ci-dessus remplace un montage analogique dans la boucle de rétroaction d'un amplificateur opérationnel, appelée « rétroaction numérique ».

Cette « rétroaction numérique » permet d'augmenter les contraintes du filtre, de l'initialiser numériquement pour qu'il soit plus rapide, d'atténuer un bruit ambiant dont la bande passante évolue dans le temps et de réaliser un multiplexage temporel à haute fréquence de filtres à basse fréquence de coupure.

Le dispositif est un système d'imagerie BCG qui comprend les éléments suivants :

- Un capteur surfacique de la pression corporelle,
- Une unité d'acquisition avec multiplexage temporel d'un filtre et d'un amplificateur,
- Un logiciel de traitement de signal numérique.

Le capteur surfacique de la pression corporelle est utilisé habituellement pour mesurer le confort d'un individu en position allongée ou assise et/ou les risques d'escarres. Il est généralement composé de plusieurs modules de taille 35x40 cm à 50x100 cm, avec des résolutions allant de 0.5 à 1 capteur/cm2, soit N = 1024 à 4096 capteurs par module. Exemple des « Body Pressure Measurement Systems » (en français : « systèmes de mesure de pression corporelle ») de la société Tekscan (marque déposée).

Une unité d'acquisition est mise en oeuvre par module, c'est-à-dire pour plus d'un millier de capteurs de pression.

Le filtre, linéaire ou non linéaire, est paramétrable par sa bande passante, largeur de transition, atténuation maximale en bande passante et atténuation minimale en bande coupante. L'amplificateur a un gain paramétrable. Le filtre et l'amplificateur peuvent être paramétrés en temps réel, par exemple pour amplifier un signal ou atténuer un bruit dont les bandes passantes évoluent dans le temps.

Soit fs la fréquence d'échantillonnage du signal. Pour mesurer la fonction cardiaque, on configure la bande passante du filtre à [1 ; fs / 2] et le gain de 7 à 40 dB (x5 à x100). Pour mesurer la fonction respiratoire, on configure la bande passante du filtre à [0,1 ; 1] et le gain de 7 à 40 dB. Pour mesurer les deux fonctions simultanément, on configure la bande passante du filtre à [0,5 ; fs / 2] et le gain de 7 à 40 dB. Dans les deux cas, il faut supprimer la composante continue du signal afin de ne pas faire saturer l'amplificateur (couplage AC).

Le convertisseur analogique numérique comporte un filtre anti-repliement de fréquence de coupure fs / 2.

La fréquence d'échantillonnage du signal fs est comprise entre 128 et 512 Hz, la fréquence du multiplexeur est donc N x fs, d'au moins 130 kHz pour atteindre la résolution spatio-temporelle minimale (fs = 128 Hz et N = 1024 capteurs) jusqu'à 2 MHz pour la résolution spatio-temporelle maximale (fs = 512 Hz et N = 4096 capteurs).

Enfin la résolution du quantificateur est de 8 à 16 bits, typiquement 12 bits.

Afin de multiplexer rapidement un filtre avec une bande passante aussi lente, on utilise un amplificateur opérationnel avec une boucle de rétroaction numérique.

Un avantage du montage illustré en figure 2, dans lequel la bande passante du filtre est soustraite du signal Vin par l'amplificateur, est que le filtre est un filtre numérique exécuté sur un microcontrôleur, précédé d'un convertisseur analogique numérique et suivi d'un convertisseur numérique analogique. Il peut être d'un ordre quelconque, à réponse impulsionnelle finie ou infinie, linéaire ou non linéaire, ou encore adaptatif.

De plus, l'état du filtre peut être initialisé de telle sorte que l'amplificateur sature moins longtemps, comme si on préchargeait le condensateur d'un filtre analogique à sa valeur d'équilibre.

Enfin, l'état et la sortie de ce filtre peuvent être stockés à chaque pas d'échantillonnage dans une mémoire : il peut donc être multiplexé temporellement, même si la fréquence est beaucoup plus grande que la fréquence de coupure du filtre. De plus, le filtre numérique dans la boucle de rétroaction permet de supprimer les bandes de fréquence du bruit afin d'éviter que ce dernier ne fasse saturer l'amplificateur.

Le logiciel de traitement de signal numérique peut être embarqué dans la carte d'acquisition pour traitement en temps réel ou sur ordinateur pour post-traitement. Il interpole les mesures pour augmenter numériquement la résolution de l'image et permet au clinicien de visualiser les fonctions cardiaque et respiratoire, et quantifie ces fonctions (fréquences cardiaque et respiratoire, trajectoire de l'onde de pouls, etc.).

Les avantages de cet appareil sont multiples. En milieu hospitalier, il permet de réaliser des examens des fonctions cardiaques et respiratoires simplement et rapidement, en étant intégré directement dans le lit. À domicile ou dans des contextes non médicaux, il permet de réaliser de la surveillance court et long terme des données vitales (lit, chaise, siège, vêtement).

Il possède les mêmes avantages que les systèmes de ballistocardiographie de type connu :

- il est peu sensible aux couches de vêtements et de draps comparé à d'autres systèmes de mesure, par exemple par ultra-son Doppler ou caméra CCD.
- il permet de mesurer les fonctions cardiaque et respiratoire simultanément.

Il a d'autres avantages que les systèmes de ballistocardiographie connus :

- il effectue une mesure surfacique et de haute résolution (>1000 points),
- il est plus robuste grâce à l'atténuation active du bruit et donc plus facilement utilisable en environnement bruité,
- il est plus rapide grâce à l'initialisation du filtre numérique.

De plus, son unité d'acquisition est simple, compacte et de faible coût.

Dans des modes de réalisation, l'amplificateur d'instrumentation utilisé est un AD623AN (marque déposée) dont le gain est ajustable grâce à une résistance. Dans des expérimentations, deux gains sont utilisés : un gain unitaire (absence de résistance, circuit ouvert) et un gain x10 (résistance d'environ dix kΩ). Les résultats présentés ci-dessous sont validés pour un gain x100 avec une alimentation stabilisée et un circuit présentant un plan de masse adéquat.

Dans les figures 8 et suivantes, on décrit un traitement de signaux issus d'accéléromètres. Hormis les unités et valeurs, ces signaux sont similaires aux signaux issus de capteurs de pression, avec en particulier, une composante continue, du bruit et des signaux physiologiques à extraire.

Dans des exemples d'implémentation, la sortie de l'accéléromètre est filtrée sur la carte Nucléo (marque déposée), grâce à la librairie MBED-DSP et en particulier les fonctions arm_fir_f32() et arm_biquad_cascade_df1_f32().

Conception des filtres

Deux types de filtres sont étudiés :

- les filtres FIR
- les filtres IIR.

Ils sont conçus avec la librairie scipy.signal de Python.

Le filtre FIR est d'ordre numTaps-1 et constitué de numTaps coefficients {bi}.

$$y[n] = b[0] * x[n] + b[1] * x[n-1] + b[2] * x[n-2] + ... + b[numTaps-1] * x[n-numTaps+1]$$

Le filtre FIR est calculé par la méthode des fenêtres, avec une fenêtre de Kaiser optimale. Celle-ci est déterminée avec comme contrainte une atténuation de 40 dB dans la bande passante et une transition de l'ordre de 0.1 Hz (fréquence de Nyquist 128 Hz):

numtaps,bêta = scipy.signal.kaiserord(40,0.1/128)

Le nombre de coefficients de cette fenêtre optimale s'élève à 5716. Avec les contraintes de bande passante (fréquence de coupure à 0.05 Hz) on obtient :

$$b = $$

$$\text{scipy.signal.firwin(numtaps=numtaps,cutoff=0.05,Windows=('kaiser',bêta),pass\_zero=True,}$$

$$\text{fs=256).}$$

Le filtre IIR biquadratique, Butterworth du premier ordre, est d'ordre deux et contient un dénominateur. Pour une largeur de transition et une atténuation équivalentes, l'ordre du filtre IIR est beaucoup moins élevé que pour le filtre FIR précédent, mais il peut être moins stable.

$$y[n] = b0 * x[n] + b1 * x[n-1] + b2 * x[n-2] + a1 * y[n-1] + a2 * y[n-2]$$

Il est obtenu par les fonctions suivantes :

$$b,a = \text{butter}(2,0.05/(fs/2));$$

$$\text{pCoeffs} = \text{concatenate}((b,-a[1:]));$$

On note que le filtre de Butterworth du premier ordre est intéressant à étudier, car il s'agit de l'équivalent numérique du filtre analogique réalisé dans les circuits électroniques de couplage AC (en courant alternatif) : un condensateur suivi d'une résistance, avec éventuellement un amplificateur opérationnel pour résoudre des questions d'impédances ou pour ajouter un gain.

Dans un filtre IIR biquadratique en cascade, on met en série des sections du filtre IIR précédent.

Comme exposé ci-dessus, les coefficients 64 bits calculés sur l'ordinateur doivent, dans des modes de réalisation, être quantifiés en 32 bits, et la réponse du filtre varie énormément, car les marges de stabilités sont faibles. En conséquence, certains filtres biquadratiques conçus sous l'architecture 64 bits sont instables sous 32 bits.

On note que le nombre de coefficients est beaucoup moins élevé que pour un filtre FIR donc le nombre d'opérations est beaucoup plus faible, ce qui est favorable à la vitesse de traitement des signaux. On peut aussi améliorer la structure du filtre pour diminuer encore le nombre d'opérations (structure de type II) ou pour diminuer l'influence de la quantification des coefficients (structure de Gold & Rader).

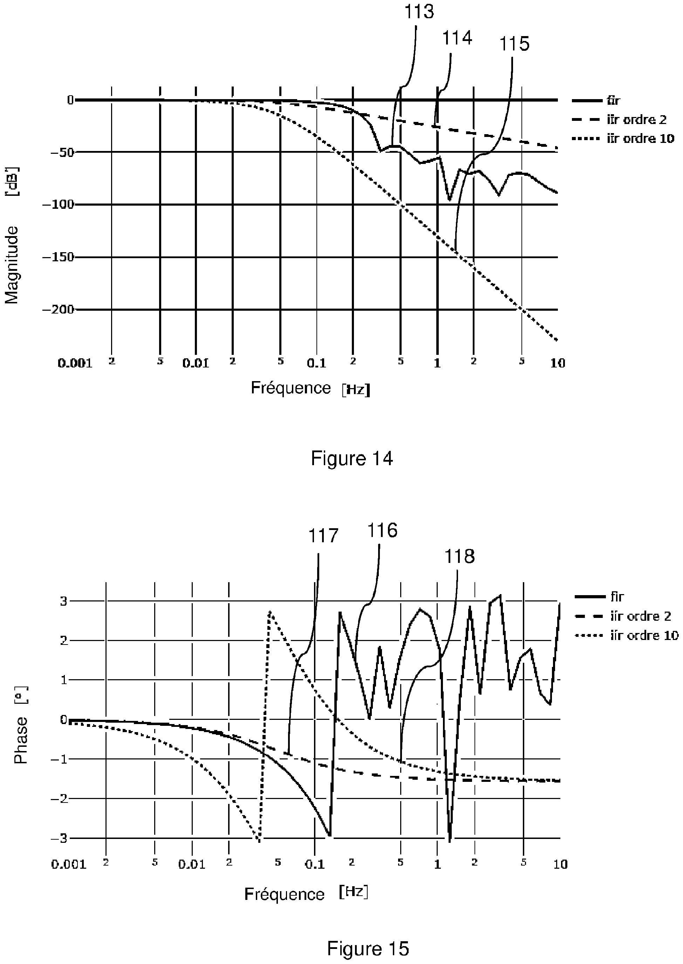

Les figures 14 et 15 représentent, en amplitudes et en phases, les réponses fréquentielles de différents filtres. Les courbes 113 et 116 représentent la réponse d'un filtre FIR. Les courbes 114 et 117 représentent la réponse d'un filtre IIR d'ordre deux. Les courbes 115 et 118 représentent la réponse d'un filtre IIR d'ordre dix. On voit tout l'intérêt de passer par des filtres numériques (transition plus abrupte, atténuation plus importante). La réponse du filtre IIR d'ordre deux est équivalente à celle d'un filtre passe-bas analogique de premier ordre.

On note que, dans d'autres modes de réalisation, on choisit d'autres filtres, par exemple des filtres non linéaires (filtre médian, ondelettes ...) et également pour d'autres utilisations que le couplage AC.

Concernant la saturation, l'amplificateur AD623 est alimenté sous 0 - 5 V donc sa tension de sortie, en cas de saturation, peut excéder la tension maximale du convertisseur analogique numérique. Il convient soit d'ajouter une saturation hardware sur la sortie de l'amplificateur, soit une saturation software sur la sortie du convertisseur numérique analogique. Cette deuxième voie présente des avantages en termes de simplicité de mise en oeuvre :

```
float expectedOutput = (rawSignal - filteredSignal)*analogGain+Vref;
if (expectedOutput > 3.3){
feedback.write((rawSignal+(Vref-3.3)/analogGain)/3.3);
} else if (expectedOutput<0){
feedback.write((rawSignal+Vref/analogGain)/3.3);
} else {
feedback.write(filteredSignal/3.3);
}
```

Pour que la saturation soit correcte, il convient de mesurer précisément la tension Vref ainsi que le gain : avec un voltmètre pour le premier (1,69 V) et avec la valeur de résistance pour le second (11,9 k$\Omega$ soit un gain x9.4).

L'état du filtre est usuellement initialisé à zéro, c'est-à-dire que toutes les valeurs x[n] et y[n] pour lesquelles n<0 sont nulles. Le signal est alors causal.

Le capteur a une position de repos qui est connue ou peut être estimée. Pour un accéléromètre en ballistocardiographie, on suppose qu'il est posé à plat sur le matelas qui est lui-même à l'horizontale : l'axe z voit donc +1g en valeur de courant continu. D'après la fiche technique de l'accéléromètre LIS344ALH la valeur, la valeur de courant continu de sortie de l'accéléromètre est donc 2,31 V. On peut initialiser l'état du filtre en imposant que les valeurs x[n] et y[n] valent 2,31 V pour numTaps<n<0.

Pour d'autres capteurs, on peut aussi réaliser un premier filtre passe-bas rapide (fréquence de coupure plus élevée) pour estimer la valeur de courant continu de l'accéléromètre et remplir le second long filtre avec ces valeurs.

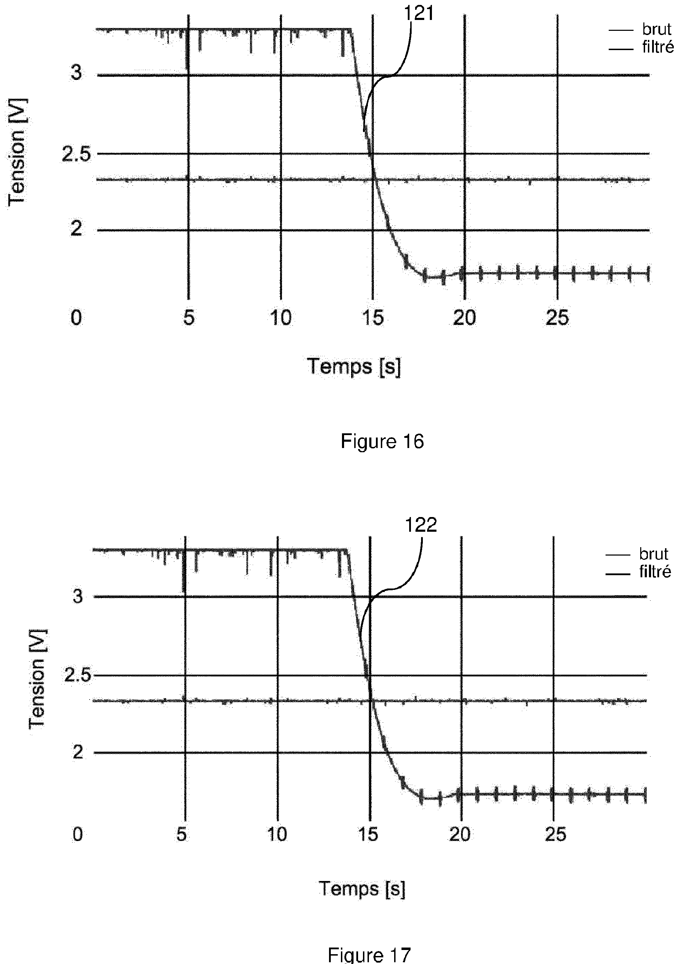

On montre, en figures 16 à 19, la réponse temporelle du filtre et de l'amplificateur dans chacune des configurations énoncées précédemment.

La figure 16 représente la réponse 121 d'un filtre FIR. On observe bien l'amplification des impulsions (elles sont cachées dans le bruit du signal brut, en dessous des limites de résolution de l'ADC), mais le filtre met une certaine durée à se charger, ce qui inhérent au fait que la fréquence de coupure est très faible. C'est également vrai pour le filtre analogique et amplifié lorsqu'on augmente le gain de l'amplificateur.

La figure 17 représente la réponse 122 d'un filtre IIR 1 section. Ce filtre a un comportement identique à celui d'un circuit passe-haut analogique du premier ordre.

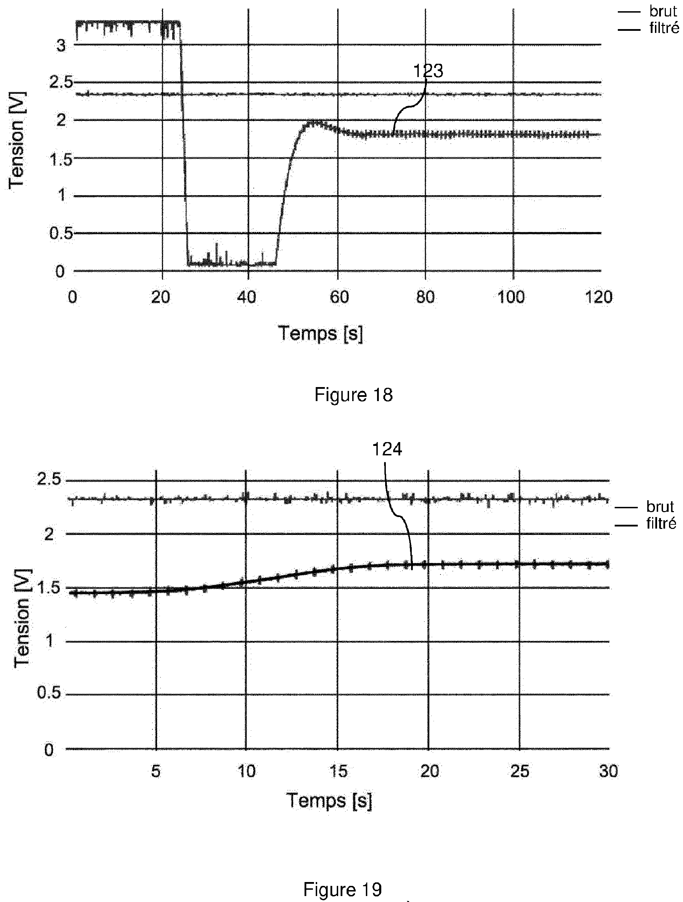

La figure 18 représente la réponse 123 d'un filtre IIR 5 sections. L'échelle de temps est beaucoup plus longue, car il y a plus de coefficients. Il n'y a pas nécessairement beaucoup d'intérêt à augmenter l'ordre du filtre IIR.

La figure 19 représente la réponse 124 d'un filtre FIR avec initialisation forcée. On observe logiquement une absence de la saturation lorsque le filtre FIR est correctement initialisé. C'est une manière de « précharger » le filtre. Une légère oscillation apparaît, ce qui correspond au fait que la valeur de la composante continue fournie pour l'initialisation du filtre est légèrement différente de la valeur de la composante continue réelle de l'accéléromètre.

On a le même résultat d'absence de la saturation lorsque le filtre est correctement initialisé avec les filtres IIR.

La présente invention s'applique, notamment :

- à la surveillance de patients pendant une opération chirurgicale et/ou
- à la visualisation de fonctions vitales, par exemple visualisation de l'onde de pouls et de la contraction des ventricules.

**Revendications**

1. Dispositif (30) de ballistocardiographie, **caractérisé en ce qu'**il comporte :

   - un support (31) pour une personne,
   - une pluralité de capteurs (32 à 35) de pression ou d'accélération fournissant, chacun, un signal analogique représentatif d'une pression ou d'une accélération mesurée en un point du support ou sur le corps de la personne,
   - un multiplexeur (41) configuré pour recevoir une pluralité de signaux analogiques en provenance de capteurs et pour fournir, en sortie, un signal successivement représentatif des signaux en entrée et
   - un amplificateur opérationnel (52) dont une entrée (57) est reliée à la sortie du multiplexeur et muni, sur son autre entrée, d'un filtre mixte comportant un convertisseur analogique numérique (53) convertissant le signal sortant du multiplexeur en données numériques, un filtre numérique (54) agissant sur les données numériques et un convertisseur numérique analogique (55) convertissant les données numériques filtrées en un signal analogique injecté sur cette autre entrée (58) de l'amplificateur opérationnel.

2. Dispositif (30) selon la revendication 1, dans lequel le filtre numérique (54) est une cascade (60) de filtres biquadratiques (61, 62).

3. Dispositif (30) selon l'une des revendications 1 ou 2, dans lequel la fréquence d'échantillonnage du convertisseur analogique numérique (53) est égale au produit du nombre de capteurs par un nombre compris entre 128 et 512.

4. Dispositif (30) selon l'une des revendications 1 à 3, dans lequel la pluralité de capteurs (32 à 35) constituent au moins une matrice de capteurs de pression.

5. Dispositif (30) selon l'une des revendications 1 à 4, dans lequel le gain de l'amplificateur (52) est supérieur à 10.

6. Dispositif de surveillance d'un patient pendant une opération chirurgicale, qui comporte un dispositif (30) selon l'une des revendications 1 à 5.

7. Application d'un dispositif (30) selon l'une des revendications 1 à 5 à la visualisation de l'onde de pouls et de la contraction des ventricules.

8. Procédé de ballistocardiographie mettant en oeuvre un dispositif (30) selon l'une des revendications 1 à 5, qui comporte les étapes suivantes :

   - une pluralité de captures de pression ou d'accélération en différents points (32 à 35) d'un support (31) d'une personne ou sur le corps de la personne, fournissant, pour chaque point de capture, un signal analogique représentatif d'une pression ou d'une accélération mesurée en un point du support ou sur le corps de la personne,
   - un multiplexage de la pluralité de signaux analogiques et
   - une amplification du signal multiplexé auquel est soustrait un signal filtré par un filtre mixte comportant un convertisseur analogique numérique (53) convertissant le signal multiplexé en données numériques, un filtre numérique (54) agissant sur les données numériques et un convertisseur numérique analogique (55) convertissant les données numériques filtrées en un signal analogique.

9. Procédé selon la revendication 8, qui comporte, de plus, une étape d'estimation de la composante continue et une étape d'initialisation du filtre (54) avec la composante continue estimée.

10. Procédé selon l'une des revendications 8 ou 9, qui comporte, de plus :

    - une étape de détection d'une vibration dans le signal amplifié,
    - une étape de segmentation du signal de vibration,
    - une étape de transformation, dans le domaine fréquentiel, de segments du signal,
    - une étape d'obtention d'un maximum du signal, dans le domaine fréquentiel,
    - une étape de configuration d'un filtre coupe-bande comportant le maximum obtenu et
    - une étape de filtrage du signal amplifié avec le filtre coupe-bande.

**Patentansprüche**

1. Ballistokardiographievorrichtung (30), **dadurch gekennzeichnet, dass** sie umfasst:

   - eine Halterung (31) für eine Person,
   - eine Vielzahl von Druck- oder Beschleunigungssensoren (32 bis 35), die jeweils ein analoges Signal liefern, das für einen Druck oder eine Beschleunigung repräsentativ ist, der/die an einem Punkt der Halterung oder am Körper der Person gemessen wird,
   - einen Multiplexer (41), der ausgelegt ist, eine Vielzahl analoger Signale von Sensoren zu empfangen und ausgehend ein Signal bereitzustellen, das nacheinander für die Eingangssignale repräsentativ ist, und
   - einen Betriebsverstärker (52), von dem ein Eingang (57) mit dem Ausgang des Multiplexers verbunden ist und der an seinem anderen Eingang mit einem Mischfilter ausgestattet ist, der einen Analog-Digital-Wandler (53) aufweist, der das vom Multiplexer ausgehende Signal in digitale Daten umwandelt, einen digitalen Filter (54), der auf die digitalen Daten wirkt und einen Digital-Analog-Wandler (55), der die gefilterten digitalen Daten in ein analoges Signal umwandelt, das über diesen anderen Eingang (58) des Betriebsverstärkers eingespeist wird.

2. Vorrichtung (30) nach Anspruch 1, wobei der digitale Filter (54) eine Kaskade (60) biquadratischer Filter (61, 62) ist.

3. Vorrichtung (30) nach einem der Ansprüche 1 oder 2, wobei die Abtastfrequenz des Analog-DigitalWandlers (53) gleich dem Produkt aus der Anzahl der Sensoren und einer Zahl zwischen 128 und 512 ist.

4. Vorrichtung (30) nach einem der Ansprüche 1 bis 3, wobei die Vielzahl von Sensoren (32 bis 35) mindestens eine Drucksensormatrix bilden.

5. Vorrichtung (30) nach einem der Ansprüche 1 bis 4, wobei die Verstärkung des Verstärkers (52) größer als 10 ist.

6. Vorrichtung zur Überwachung eines Patienten während eines chirurgischen Eingriffs, die eine Vorrichtung (30) nach einem der Ansprüche 1 bis 5 aufweist.

7. Anwendung einer Vorrichtung (30) nach einem der Ansprüche 1 bis 5 zur Visualisierung der Pulswelle und der Kontraktion der Ventrikel.

8. Verfahren zur Ballistokardiographie, das eine Vorrichtung (30) nach einem der Ansprüche 1 bis 5 verwendet, das die folgenden Schritte aufweist:

- eine Vielzahl von Druck- oder Beschleunigungserfassungen an verschiedenen Punkten (32 bis 35) einer Halterung (31) einer Person oder am Körper der Person stellt für jeden Erfassungspunkt ein analoges Signal bereit, das für einen Druck oder eine Beschleunigung repräsentativ ist, der/die an einem Punkt der Halterung oder am Körper der Person gemessen wird,
- eine Multiplexierung der Vielzahl analoger Signale und
- eine Verstärkung des Multiplexsignals, von dem ein von einem Mischfilter gefiltertes Signal abgezogen wird, der einen Analog-Digital-Wandler (53) umfasst, der das Multiplexsignal in digitale Daten umwandelt, einen digitalen Filter (54), der auf die digitalen Daten wirkt, und einen Digital-Analog-Wandler (55), der die gefilterten digitalen Daten in ein analoges Signal umwandelt.

9. Verfahren nach Anspruch 8, das weiterhin einen Schritt zur Schätzung der kontinuierlichen Komponente und einen Schritt zur Initialisierung des Filters (54) mit der geschätzten kontinuierlichen Komponente aufweist.

10. Verfahren nach einem der Ansprüche 8 oder 9, das weiterhin aufweist:

- einen Erkennungsschritt einer Vibration im verstärkten Signal,
- einen Segmentierungsschritt des Vibrationssignals,
- einen Transformationsschritt von Signalsegmenten im Frequenzbereich,
- einen Schritt zum Erhalten eines Maximums des Signals im Frequenzbereich,
- einen Konfigurationsschritt eines Bandsperrfilters, der das erhaltene Maximum aufweist und
- einen Filterschritt des verstärkten Signals mit dem Bandsperrfilter.

**Claims**

1. Ballistocardiography device (30), **characterized in that** it comprises:

- a support (31) for a person;
- a plurality of pressure or acceleration sensors (32 to 35) each providing an analogue signal representative of a pressure or an acceleration measured at a point on the support or on the body of the person;
- a multiplexer (41) configured to receive a plurality of analogue signals from sensors and to output a signal successively representative of the input signals; and
- an operational amplifier (52) having one input (57) connected to the output of the multiplexer and provided, on its other input, with a mixed filter comprising an analogue-to-digital converter (53) converting the signal coming out of the multiplexer into digital data, a digital filter (54) acting on the digital data and a digital-to-analogue converter (55) converting the filtered digital data into an analogue signal that is fed into this other input (58) of the operational amplifier.

2. Device (30) according to claim 1, wherein the digital filter (54) is a cascade (60) of biquad filters (61, 62).

3. Device (30) according to one of claims 1 or 2, wherein the sampling frequency of the analogue-to-digital converter (53) is equal to the number of sensors multiplied by a number between 128 and 512.

4. Device (30) according to one of claims 1 to 3, wherein the plurality of sensors (32 to 35) forms at least one matrix of pressure sensors.

5. Device (30) according to one of claims 1 to 4, wherein the gain of the amplifier (52) is greater than 10.

6. Surveillance device for a patient during a surgical operation, comprising a device (30) according to one of claims 1 to 5.

7. Application of a device (30) according to one of claims 1 to 5 for viewing the pulse wave and the contraction of the ventricles.

8. Ballistocardiography method utilizing a device (30) according to one of claims 1 to 5, which comprises the following steps:

    - a plurality of pressure or acceleration captures at different points (32 to 35) of a support (31) for a person providing, for each capture point, an analogue signal representative of a pressure or an acceleration measured at a point on the support or on the body of the person;
    - a multiplexing of the plurality of analogue signals; and
    - an amplification of the multiplexed signal from which is subtracted a signal filtered by a mixed filter comprising a mixed filter comprising an analogue-to-digital converter (53) converting the signal coming out of the multiplexer into digital data, a digital filter (54) acting on the digital data and a digital-to-analogue converter (55) converting the filtered digital data into an analogue signal.

9. Method according to claim 8, which also comprises a step of estimating the direct component, and a step of initializing the filter (54) with the estimated direct component.

10. Method according to one of claims 8 or 9, which also comprises:

    - a step of detecting a vibration in the amplified signal;
    - a step of segmenting the vibration signal;
    - a step of transforming segments of the signal in the frequency domain;
    - a step of obtaining a maximum of the signal in the frequency domain;
    - a step of configuring a notch filter comprising the maximum obtained; and
    - a step of filtering the amplified signal with the notch filter.

Figure 1

Figure 2

Figure 3

Figure 4

Figure 5

71    70    72

Non
quantifié — quantifié — rectifié

Magnitude [dB]

0

-5

-10

0.01    0.02    0.03    0.04    0.05  0.06 0.07 0.08 0.09 1

Phase [°]

-0.5

-1

-1.5

-2

-2.5

0.01    0.02    0.03    0.04    0.05  0.06 0.07 0.08 0.09 1

Fréquence [Hz]

Figure 6

75    76    77

— RC — FIR — IIR

Magnitude [dB]

0

-50

-100

0.001  2    5  0.01  2    5  0.1  2    5  1    2    5  10

Phase [°]

2

0

-2

0.001  2    5  0.01  2    5  0.1  2    5  1    2    5  10

Fréquence [Hz]

Figure 7

80

Figure 8

90

Accélération en entrée

Sortie d'amplificateur

Figure 9

Figure 10

Figure 11

Figure 12

Figure 13

Figure 14

Figure 15

Figure 16

Figure 17

Figure 18

Figure 19

**EP 4 120 911 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20090054742 A

**Littérature non-brevet citée dans la description**

- **POSTOLACHE O. et al.** *Vital Signs Monitoring System Based on EMFi Sensors and Wavelet Analysis*

- **RIKKY MULLER et al.** *A 0.013mm2, 5$\mu$W, DC-Coupled Neural Signal Acquisition IC with 0.5 V Supply*
- **MOHAMMAD MOIARRADI et al.** *Low-Cutoff, High-Pass Digital Filtering of Neural Signals*